(19) Europäisches Patentamt
European Patent Office
Office européen des brevets

(11) EP 4 527 883 A1

(12) **EUROPEAN PATENT APPLICATION**
published in accordance with Art. 153(4) EPC

(43) Date of publication:
**26.03.2025 Bulletin 2025/13**

(21) Application number: **23807946.1**

(22) Date of filing: **19.05.2023**

(51) International Patent Classification (IPC):
***C08L 5/00*** *(2006.01)* ***C08B 37/00*** *(2006.01)*
***C08J 3/075*** *(2006.01)* ***A61L 15/28*** *(2006.01)*
***A61L 15/60*** *(2006.01)*

(52) Cooperative Patent Classification (CPC):
**A61L 15/28; A61L 15/60; C08B 37/00; C08J 3/075; C08L 5/00**

(86) International application number:
**PCT/KR2023/006832**

(87) International publication number:
**WO 2023/224427 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.05.2022 KR 20220062381**

(71) Applicant: **LG Chem, Ltd.**
**Yeongdeungpo-gu**
**Seoul 07336 (KR)**

(72) Inventors:
- **CHO, Beom Shin**
**Daejeon 34122 (KR)**
- **LEE, Mi Yeon**
**Daejeon 34122 (KR)**
- **YUN, Hae Sung**
**Daejeon 34122 (KR)**
- **CHOI, Hyung Sam**
**Daejeon 34122 (KR)**
- **HAM, Kyung Rok**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **POLYMER MATERIAL**

(57) The present application relates to a polymer material and a use thereof. The present application can provide a polymer material having both excellent biodegradability and absorptivity by using a polysaccharide component capable of efficient cross-linking while using no cross-linking agent that can adversely affect biodegradability, or using the relevant component to the minimum. The present application can also provide a use of the polymer material.

[Figure 1]

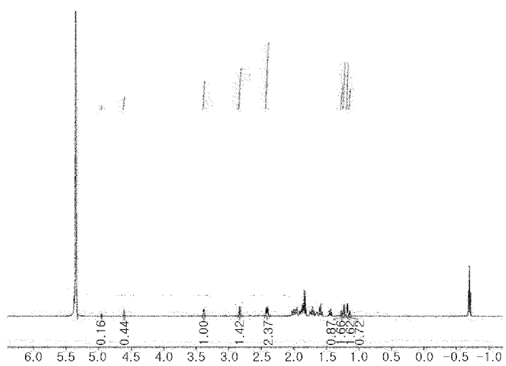


EP 4 527 883 A1

## Description

### Technical Field

This application claims the benefit of priority based on Korean Patent Application No. 10-2022-0062381 dated May 20, 2022, the disclosure of which is incorporated herein by reference in its entirety.

The present application relates to a polymer material and a use thereof.

### Background Art

A hydrogel polymer is generally defined as a cross-linked hydrophilic polymer, and is also simply called a hydrogel.

Such a polymer can be used as a material called an SAP (Super Absorbent Polymer). The SAP is a material that can absorb moisture tens to thousands of times its own weight. The SAP is used for various applications, such as sanitary products such as hygiene products or diapers, medical products, household materials, agricultural materials, horticultural materials, transportation materials, civil engineering and construction materials, materials related to electrical and electronic devices, or water treatment agents.

The most widely used hydrogel polymers used as SAPs are polymers made of vinyl-based materials such as cross-linked polyacrylic acid.

Such materials are relatively inexpensive and have excellent water absorption capacity, but cause various problems because they remain semi-permanently even after disposal.

To solve such problems, there are various attempts to manufacture the SAP from so-called biodegradable materials.

However, the materials known to date do not form the SAP with balanced physical properties. For example, the most representative physical property required for the SAP is absorption capacity, but in the SAP of a biodegradable material known to date, at least one characteristic of absorption capacity and biodegradability is not satisfactorily secured, or in some cases, both physical properties are not secured at an appropriate level.

For example, there are attempts to manufacture absorbent materials using polysaccharides. Since the polysaccharide has biodegradability, the absorbent material also has biodegradability. In this technology, for formation of the SAP, it is necessary to cross-link the polysaccharide, where an acrylate-based or vinyl-based compound is mainly used for this cross-linking. The SAP cross-linked with such a material can secure a certain degree of absorption capacity, but has a problem that biodegradability is lowered. Therefore, if the cross-linking is performed without using any cross-linking agent or while minimizing the amount used, absorption capacity and biodegradability can be secured, but considering the cross-linking efficiency of the polysaccharide, this method is not easy.

## Disclosure

### Technical Problem

The present application is intended to provide a polymer material capable of securing simultaneously excellent absorption capacity and biodegradability, and a use thereof.

### Technical Solution

Among the physical properties mentioned in this specification, when the measurement temperature and/or pressure affects the physical property value, the relevant physical property means a physical property measured at room temperature and/or normal pressure, unless specifically mentioned otherwise.

In this specification, the term room temperature is a natural temperature without heating or cooling, which may mean, for example, any one temperature in a range of about 10°C to 30°C, or a temperature of 23°C or 25°C or so.

In this specification, the term normal pressure refers to pressure when not particularly reduced or increased, and may mean a pressure of about normal atmospheric pressure, for example, about 740 mmHg to 780 mmHg.

Among the physical properties mentioned in this specification, when the measurement humidity affects the physical property value, the physical property means a physical property measured at natural humidity without special adjustment at the measured temperature and pressure, unless otherwise specified.

In this specification, unless otherwise specified, the term alkyl or alkyl group means an alkyl or alkyl group with 1 to 20 carbon atoms, 1 to 16 carbon atoms, 1 to 12 carbon atoms, 1 to 8 carbon atoms, or 1 to 4 carbon atoms. Such an alkyl or alkyl group may be linear, branched, or cyclic. Such an alkyl or alkyl group may also be optionally substituted with one or more substituents.

In this specification, unless otherwise specified, the term alkylene or alkylene group means a functional group which is connected to another object by separating two hydrogen atoms from an alkane, wherein it has a structure that the two hydrogen atoms are separated from other carbon atoms of the alkane. Such an alkylene or alkylene group may be an alkylene or alkylene group with 2 to 20 carbon atoms, 2 to 16 carbon atoms, 2 to 12 carbon atoms, 2 to 8 carbon atoms, or 2 to 4 carbon atoms. Such an alkylene or alkylene group may be linear, branched, or cyclic. Such an alkylene or alkylene group may also be optionally substituted with one or more substituents.

In this specification, unless otherwise specified, the term alkylidene or alkylidene group means a functional group which is connected to another object by separating two hydrogen atoms from an alkane, wherein it has a structure that the two hydrogen atoms are separated from one carbon atom of the alkane. Such an alkylidene or alkylidene group may be an alkylidene or alkylidene group with 1 to 20 carbon atoms, 1 to 16 carbon atoms, 1 to 12 carbon atoms, 1 to 8 carbon atoms, or 1 to 4 carbon atoms. Such an alkylidene or alkylidene group may be linear, branched, or cyclic. Such an alkylidene or alkylidene group may also be optionally substituted with one or more substituents.

In the present application, the term hydrogel polymer material means an absorbent material comprising a cross-linked polymer. In this specification, such a material may also simply be referred to as a hydrogel.

The absorbent material described herein may exhibit at least one characteristic of moisture content, centrifuge retention capacity (CRC), and absorption capacity under pressure (AUP).

In this specification, the biodegradable material means that the material can exhibit the biodegradability (measured according to KS M ISO 14851 standard) specified in this specification.

The present application relates to a polymer material. The polymer material may be the above-described absorbent material and simultaneously the biodegradable material.

**In** this specification, the term polymer material means a material comprising a polymer. The polymer may mean a substance formed by connecting two or more unitary bodies by covalent bonds. **In** one example, the polymer may mean a substance comprising a structure in which two or more unitary bodies are connected by covalent bonds, and having a molecular weight of a certain level or more. The range of the molecular weight is not limited, but the molecular weight of the polymer, in terms of weight average molecular weight (Mw), may be approximately 500 g/mol or more. The upper limit of the weight average molecular weight is not particularly limited, and for example, the weight average molecular weight of the polymer may be about 1,000,000 g/mol or less or so. The weight average molecular weight is a value measured by GPC (Gel Permeation Chromatograph) using polystyrene as a calibration standard sample.

**In** one example, the lower limit of the weight ratio of the polymer in the polymer material may be 55 wt%, 60 wt%, 65 wt%, 70 wt%, 75 wt%, 80 wt%, 85 wt%, 90 wt%, or 95 wt% or so, and the upper limit may be 100 wt%, 98 wt%, 96 wt%, 94 wt%, 92 wt%, or 90 wt% or so. The weight ratio of the polymer may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits.

The polymer of the present application may be a polysaccharide component.

In this specification, the term polysaccharide component means a component consisting of a polysaccharide in one molecule or a plurality of polysaccharides in two or more molecules. That is, the polysaccharide component comprises only polysaccharides, but the polysaccharide contained in the polysaccharide component may have two or more molecules. When the polysaccharides in two or more molecules are included, the polysaccharides may be the same or different from each other.

In this specification, the term polysaccharide has a meaning known in the industry. The polysaccharide generally refers to a polymer molecule in which two or more unitary bodies are linked by covalent bonds. In one example, the covalent bond connecting the unitary bodies may be a glycosidic bond. The polysaccharide is a polymer molecule, that is, a polymer, which may have a weight average molecular weight in the above-mentioned range.

The unitary body forming the polysaccharide may be a biomolecule composed of carbon, hydrogen, and oxygen, or composed of carbon, hydrogen, oxygen, and nitrogen. In this specification, the term biomolecule is interpreted to have the meaning generally applied in the industry. Typically, as an example of the biomolecule in the industry, monosaccharides such as glucose, galactose, fructose or xylose, disaccharides such as sucrose, lactose, maltose or trehalose, polyols such as sorbitol or mannitol, oligosaccharides such as maltodextrin, dextrin, raffinose, stachyose or fructo-oligosaccharide and/or amino sugars such as glucosamine or N-acetyl glucosamine, and the like are known, but the types of biomolecules applied in the present application are not limited to the foregoing.

If the polymer (e.g., polysaccharide component) contained in the polymer material is in a cross-linked state and has absorption capacity, the relevant polymer material may be called the hydrogel polymer material or hydrogel.

In one example, the polymer material may be in a powder state formed through a grinding process or the like.

The polymer material may comprise only the polysaccharide component as the polymer, or may further comprise other components in addition to the polysaccharide component. Within the polymer material, the polysaccharide component may exist in a cross-linked state.

In the polymer material, an example of another component that can be included together with the polysaccharide

component are not particularly limited, but may be exemplified by a cross-linking agent cross-linking the polysaccharide, or a polymer different from the polysaccharide, and the like.

In one example, the lower limit of the weight ratio of the polysaccharide component in the polymer material may be 55 wt%, 60 wt%, 65 wt%, 70 wt%, 75 wt%, 80 wt%, 85 wt%, 90 wt%, 92 wt%, 94 wt%, 96 wt%, or 98 wt% or so, and the upper limit thereof may be 100 wt%, 98 wt%, 96 wt%, 94 wt%, 92 wt%, or 90 wt% or so. The weight ratio may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits.

The ratio of the polysaccharide component in the polymer material is not particularly limited, but the higher the ratio, the greater the biodegradability of the polymer material. In the present application, as a specific type of polysaccharide component is applied as the polysaccharide component, the appropriate cross-linking can be performed while using no cross-linking agent capable of reducing biodegradability or minimizing the amount used, and the cross-linked material can simultaneously exhibit excellent absorption capacity and biodegradability as desired.

When the polymer material is an absorbent material, the polymer material may exhibit a moisture content of about 40 to 70 wt% or so. In another example, the moisture content may be 45 wt% or more, 50 wt% or more, or 55 wt% or more or so, or may also be 65 wt% or less, or 60 wt% or less or so.

The moisture content is the content of moisture contained in the polymer material relative to the total weight of the polymer to be measured, which may be calculated through the weight of the polymer material containing moisture and the weight of the dried polymer material. For example, the moisture content may be calculated through the weight loss due to evaporation of moisture in the polymer material during a process of raising the temperature of the polymer material in a crumb state through infrared heating to dry it. The drying process for measuring the moisture content may comprise raising the temperature from room temperature to about 50°C or so and then performing vacuum drying for about 6 hours or so while maintaining 50°C. The polymer material may exhibit the water content before or after cross-linking.

When the polymer material is an absorbent material, the lower limit of the centrifuge retention capacity (CRC) of the polymer material according to an EDANA (European Disposables and Nonwovens Association) method WSP 241.3 may be 12 g/g, about 13 g/g, about 14 g/g, about 15 g/g, 16 g/g, 17 g/g, or 17.5 g/g or so, and the upper limit thereof may be 60 g/g, 55 g/g, 50 g/g, 45 g/g, 40 g/g, 35 g/g, 30 g/g, 25 g/g, 20 g/g, 19 g/g, or 18 g/g or so. The centrifuge retention capacity may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. The polymer material may exhibit the centrifuge retention capacity before or after cross-linking.

When the polymer material is an absorbent material, the lower limit of the absorption capacity under pressure (AUP) of the polymer material at 0.7 psi according to an EDANA (European Disposables and Nonwovens Association) method WSP 242.3 may be 4 g/g, 6 g/g, 8 g /g, 10 g/g, 12 g/g, 14 g/g, 16 g/g, 18 g/g, 20 g/g, 22 g/g, 24 g/g, or 26 g/g or so, and the upper limit thereof may be 40 g/g, 38 g/g, 36 g/g, 34 g/g, or about 32 g/g or so. The absorption capacity under pressure may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. The polymer material may exhibit the absorption capacity under pressure before or after cross-linking.

If the polymer material exhibits at least one characteristic of the moisture content, centrifuge retention capacity, and absorption capacity under pressure as described above, the relevant material may be defined as an absorbent material.

The polymer material may exhibit excellent biodegradability. For example, the polymer material may be the absorbent material and simultaneously the biodegradable material. For example, the lower limit of the biodegradability of the polymer material may be 60%, 62%, 64%, 66%, 68%, 70%, 72%, 74%, 76%, 78%, 80%, 82%, 84%, 86%, or 88% or so, and the upper limit thereof may be 100%, 98%, 96%, 94%, 92%, 90%, or 88% or so. The biodegradability may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. The polymer material may exhibit the biodegradability before or after cross-linking. The biodegradability is identified according to KS M ISO 14851 standard. The biodegradability appears differently depending on a measurement method. For example, the KS M ISO 14855-1 is a method of measuring biodegradability through carbon dioxide under a composting condition, where a higher level of biodegradability is measured for the same material compared to other measurement methods, but in the actual environment, it does not represent the biodegradability of the material well. The KS M ISO 14851 standard is a method of measuring aerobic biodegradability of a polymer material in an aqueous solution medium (measurement of oxygen consumption by a closed respiratory system), where the biodegradability of this method can well reflect the biodegradation characteristics of the material in the actual environment.

The polymer material of the present application can exhibit excellent absorptivity and biodegradability simultaneously as described above.

Such a material can be secured by applying a polysaccharide component to be described below as the polysaccharide component. In general, the polysaccharide-based polymer material has excellent biodegradability, but has low water absorption capacity and low cross-linking efficiency for application as an absorbent material. Therefore, in general, to form an absorbent material using a polysaccharide-based material, it is obtained by blending other components with the polysaccharide without applying only the polysaccharide. However, in the present application, a polysaccharide component in which a specific type of functional group is introduced at a predetermined ratio is applied as the polysaccharide component. Such a polysaccharide component is capable of cross-linking, and self-cross-linking as well, which is described below, while using no cross-linking agent that may adversely affect biodegradability or applying a minimal amount. Such a polysaccharide component can simultaneously exhibit excellent absorption capacity and biodegradability after cross-linking. The cross-linking as mentioned in this specification means the initial cross-linking to cross-link the polysaccharide component without cross-linking. That is, the polysaccharide component is capable of further cross-linking, if necessary, after the cross-linking or self-cross-linking performed while using no cross-linking agent that may adversely affect the biodegradability or applying a minimum amount. Upon such further cross-linking, a cross-linking agent such as an acrylate series may also be used.

As described above, the polysaccharide is a polymeric substance in which two or more unitary bodies are linked by covalent bonds such as glycosidic bonds, and the representatively known polysaccharide includes starch, dextrin, or chitosan, and the like.

In one example, when the polysaccharide is starch, the weight ratio (amylose: amylopectin) of amylose and amylopectin in the relevant starch may be in a range of about 1:99 to 50:50 or so. In this case, the functional group to be described below may be introduced into either amylose or amylopectin, or may be introduced into both. The reaction of introducing the functional group, which is described below, can occur in both amylose and amylopectin, but more efficient modification may be possible when the content of amylopectin is greater than the content of amylose.

For example, the starch may have a gelatinization temperature in a range of about 50°C to 90°C and a peak viscosity (BU) in a range of 50 to 1000.

As the starch, any known starch may be applied without special limitation, and for example, one or two or more types selected from potato starch, corn starch, rice starch, wheat starch, tapioca starch, and polymer starch may be applied.

Such a polysaccharide may comprise at least two or more monosaccharide unitary bodies linked by covalent bonds (e.g., glycosidic bonds). At this time, the monosaccharide unitary body may be exemplified by the above-described biomolecule, and may be specifically exemplified by glucose, galactose, fructose, xylose, glucosamine, or N-acetylglucosamine, and the like, but is not limited thereto.

Among the unitary bodies included in the polysaccharide, at least one may be a modified monosaccharide unitary body. The modified monosaccharide unitary body means a unitary body into which a functional group that has not been present in the original unitary body is introduced through chemical treatment.

In one example, the functional group introduced into the modified monosaccharide unitary body may be represented, for example, by Formula 1 below.

[Formula 1]

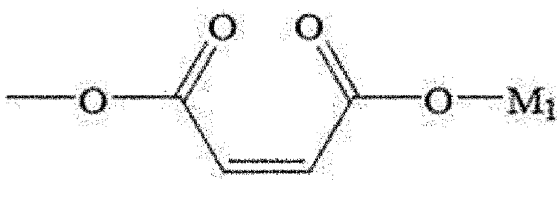

In Formula 1, $M_1$ is hydrogen or a metal. In Formula 1, when $M_1$ is a metal, the $O-M_1$ bond is an ionic bond.

The functional group of Formula 1 may be introduced into the unitary body by bonding the oxygen atom on the left side of Formula 1 above to the skeleton of the unitary body.

In the functional group of Formula 1, the carbon-carbon double bond may exist in the polymer in a state where the relevant double bond is maintained, or may exist in the polymer in a state where the double bond participates in cross-linking.

In Formula 1, $M_1$ is hydrogen or a metal. The type of metal is not particularly limited, which may usually be an alkali metal such as lithium, sodium, potassium, or cesium. Such a metal may exist in a cationic form.

In the polymer, a functional group where $M_1$ in Formula 1 above is hydrogen and a functional group where $M_1$ is a metal may also exist simultaneously.

The functional group may be introduced by reacting the polysaccharide with an unsaturated dicarboxylic acid or

an anhydride thereof and substituting a hydroxy group, and the like present in the unitary body of the polymer with the functional group. An example of the dicarboxylic acid or anhydride thereof may be exemplified by maleic acid or maleic anhydride, but is not limited thereto, and salts of maleic acid may also be applied.

A substitutional rate of the functional group of Formula 1 in the polysaccharide may be within a predetermined range. The lower limit of the substitutional rate may be 10%, 15%, 20%, 25%, 30%, 35%, 40%, 45%, 50%, 55%, 60%, 65%, 70%, 75%, 80%, 85%, 90%, or 95% or so, and the upper limit thereof may be 300%, 295%, 290%, 285%, 280%, 275%, 270%, 265%, 260%, 255%, 250%, 245%, 240%, 235%, 230%, 225%, 220%, 215%, 210%, 205%, 200%, 195%, 190%, 185%, 180%, 175%, 170%, 165%, 160%, 155%, 150%, 145%, 140%, 135%, 130%, 125%, 120%, 115%, 110%, 105%, 100%, 95%, 90%, 85%, 80%, 75%, 70%, 65%, 60%, 55%, 50%, 45%, or 40% or so. The substitutional rate may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. Within the range of the substitutional rate, cross-linking of the polysaccharide component can proceed effectively, and the physical properties (for example, absorption capacity and/or biodegradability, etc.) of the resulting polymer material can be stably secured.

In this specification, the substitutional rate is a value indicating how much the hydroxy groups present in the respective unitary bodies included in the polysaccharide are replaced with predetermined functional groups (e.g., functional groups of Formula 1 above), and is an average value of substituted degrees of the respective unitary bodies present in the polysaccharide. For example, if the unit body is a grape sugar (glucose) unit, there are three hydroxy groups in the unit before modification, and therefore, if all the hydroxy groups are substituted with the functional groups of Formula 1, the substitutional rate for the relevant unit is 300%. However, the substitutional rate of the polysaccharide is the average value of the substituted degrees of the respective units present in the polysaccharide, so that for example, when in a polysaccharide containing 5 grape sugar (glucose) units, the substitutional rates of the respective units are 100%, 0%, 200%, 300% and 100%, the substitutional rate of the polysaccharide is 140%, which is the average value. Such a substitutional rate can be identified through $^{1}$H NMR analyses of the polysaccharide. That is, since the hydroxy groups and substituted functional groups present in the polysaccharide can be quantified through the $^{1}$H NMR analysis, the substitutional rate can be identified, and if necessary, the substitutional rate can be calculated considering the $^{1}$H NMR analysis results of the polysaccharide before modification. In this way, the method of quantifying functional groups through the $^{1}$H NMR analysis is known.

In one example, the unitary body containing the functional group of Formula 1 may be a unitary body represented by Formula 2 below.

[Formula 2]

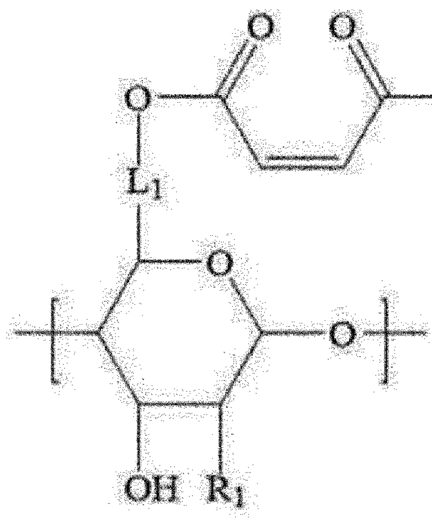

In Formula 2, $R_1$ may be a hydroxy group, an amino group, or an alkylcarbonylamino group. In Formula 2, $L_1$ may be an alkylene group or an alkylidene group. In Formula 2, $M_1$ may be hydrogen or a metal.

If $M_1$ in Formula 2 is the metal, the bond of O-$M_1$ in Formula 2 may be an ionic bond.

The specific type of the metal of $M_1$ in Formula 2 is as described in Formula 1.

In Formula 2, the specific type of the alkyl, alkylene group, or alkylidene group is the same as defined at the opening of this specification.

As described in Formula 1, the carbon-carbon double bond of the functional group in Formula 2 may exist in the polymer in a state where the double bond is maintained, or may exist in the polymer in a state where the double bond participates in cross-linking.

In the polymer, a functional group where $M_1$ in Formula 2 above is hydrogen and a functional group where $M_1$ is a metal may also exist simultaneously.

In Formula 2, when $R_1$ is a hydroxy group, the unitary body may usually be a case derived from a so-called grape sugar unitary body; when $R_1$ is an amino group, the unitary body may usually be a case derived from a so-called glucosamine unitary body; and when $R_1$ is an alkylcarbonylamino group, the unitary body may represent a case derived from N-acetylglucosamine.

In one example, the functional group introduced into the modified monosaccharide unitary body may also be, for example, a functional group represented by Formula 3 below.

[Formula 3]

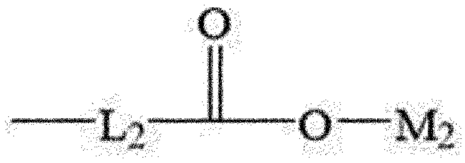

In Formula 3, $L_2$ may be an alkylene group or an alkylidene group. In Formula 3, $M_2$ may be hydrogen or a metal. In Formula 3, when $M_2$ is the metal, the O-$M_2$ bond may be an ionic bond.

In Formula 3, the specific type of the alkylene group or alkylidene group is the same as defined at the opening of this specification.

The functional group of Formula 3 may be introduced into the unitary body as $L_2$ on the left side in Formula 3 above is bonded to the skeleton of the unitary body.

In Formula 3, $M_2$ is hydrogen or a metal. The type of the metal is not particularly limited, which may usually be an alkali metal such as lithium, sodium, potassium, or cesium.

In the polysaccharide, a functional group where $M_2$ in Formula 3 above is hydrogen and a functional group where $M_2$ is a metal may also exist simultaneously.

The functional group can be introduced by reacting the polysaccharide with acetic acid chloride such as chloroacetate and substituting the hydroxy group present in the unitary body of the polymer with the functional group.

The lower limit of the substitutional rate of the functional group of Formula 3 in the polysaccharide may be 10%, 15%, 20%, 25%, 30%, 35%, 40%, 45%, 50%, 55%, 60%, 65%, 70%, 75%, 80%, 85%, 90%, or 95% or so, and the upper limit may be 300%, 295%, 290%, 285%, 280%, 275%, 270%, 265%, 260%, 255%, 250%, 245%, 240%, 235%, 230%, 225%, 220%, 215%, 210%, 205%, 200%, 195%, 190%, 185%, 180%, 175%, 170%, 165%, 160%, 155%, 150%, 145%, 140%, 135%, 130%, 125%, 120%, 115%, 110%, 105%, 100%, 95%, 90%, 85%, 80%, 75%, or 70% or so. The substitutional rate may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. Within the range of the substitutional rate, cross-linking of the polysaccharide component can proceed effectively, and the physical properties (for example, absorption capacity and/or biodegradability, etc.) of the resulting polymer material can be stably secured. The definition of the substitutional rate is the same as defined for the functional group in Formula 1, which can be identified through the $^1$H NMR analysis of the polysaccharide equally.

In one example, the unitary body containing the functional group of Formula 3 above may be a unitary body represented by Formula 4 below.

[Formula 4]

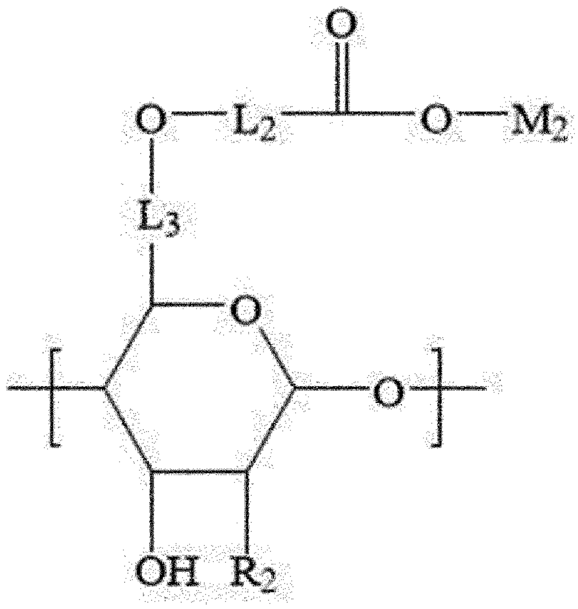

In Formula 4, $R_2$ may be a hydroxy group, an amino group, or an alkylcarbonylamino group, $L_2$ and $L_3$ may each independently be an alkylene group or an alkylidene group, and $M_2$ may be hydrogen or a metal.

In Formula 4, when $M_2$ is the metal, the O-$M_2$ bond may be an ionic bond. The specific type of the metal of $M_2$ in Formula 4 is as described in Formula 3.

In Formula 4, the specific type of the alkyl, alkylene group, or alkylidene group is the same as defined at the opening of this specification.

In the polysaccharide, a functional group in which $M_2$ in Formula 4 above is hydrogen and a functional group in which $M_2$ is a metal may also exist simultaneously.

In the polysaccharide component included in the polymer material, the functional group of Formula 1 above and the functional group of Formula 3 above may also exist simultaneously. For example, in the polysaccharide in one molecule, the functional group of Formula 1 above and the functional group of Formula 3 above may exist simultaneously, or a polysaccharide in which the functional group of Formula 1 above exists and a polysaccharide in which the functional group of Formula 3 above exists may also be included in the polymer material together.

Therefore, in the polysaccharide contained in the polymer material, the unitary body of Formula 2 above and the unitary body of Formula 4 above may also exist simultaneously. For example, in the polysaccharide in one molecule, the unitary body of Formula 2 above and the unitary body of Formula 4 above may exist simultaneously, or a polysaccharide in which the unitary body of Formula 2 above exists and a polysaccharide in which the unitary body of Formula 4 above exists may also be included in the polymer material together.

In one example, the polymer material may comprise a polysaccharide component including a modified monosaccharide unitary body (e.g., a modified monosaccharide unitary body of Formula 2 above) containing the functional group of Formula 1 above, and a modified monosaccharide unitary body (e.g., a modified monosaccharide unitary body of Formula 4 above) containing the functional group of Formula 3 above.

In the case where the polysaccharide in one molecule simultaneously contains the modified monosaccharide unitary body containing the functional group of Formula 1 and the modified monosaccharide unitary body containing the functional group of Formula 3 as above, the lower limit of the substitutional rate of the functional group of Formula 1 above in the polysaccharide may be 10%, 15%, 20%, 25%, 30%, or 35% or so, and the upper limit thereof may be 300%, 295%, 290%, 285%, 280%, 275%, 270%, 265%, 260%, 255%, 250%, 245%, 240%, 235%, 230%, 225%, 220%, 215%, 210%, 205%, 200%, 195%, 190%, 185%, 180%, 175%, 170%, 165%, 160%, 155%, 150%, 145%, 140%, 135%, 130%, 125%, 120%, 115%, 110%, 105%, 100%, 95%, 90%, 85%, 80%, 75%, 70%, 65%, 60%, 55%, 50%, 45%, or 40% or so. The substitutional rate may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. Within the range of the substitutional rate, cross-linking of the polysaccharide can proceed effectively, and the physical properties (for example, absorption capacity and/or biodegradability, etc.) of the resulting polymer material can be stably secured.

In the case where the polysaccharide in one molecule simultaneously contains the modified monosaccharide unitary body containing the functional group of Formula 1 and the modified monosaccharide unitary body containing the functional group of Formula 3, the lower limit of the substitutional rate of the functional group of Formula 3 above may be 10%, 15%, 20%, 25%, 30%, 35%, 40%, 45%, 50%, 55%, 60%, or 65% or so, and the upper limit thereof may be 300%, 295%, 290%, 285%, 280%, 275%, 270%, 265%, 260%, 255%, 250%, 245%, 240%, 235%, 230%, 225%, 220%, 215%, 210%, 205%, 200%, 195%, 190%, 185%, 180%, 175%, 170%, 165%, 160%, 155%, 150%, 145%, 140%, 135%, 130%, 125%, 120%, 115 %, 110%, 105%, 100%, 95%, 90%, 85%, 80%, 75%, or 70% or so. The substitutional rate may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. Within the range of the substitutional rate, cross-linking of the polysaccharide can proceed effectively, and the physical properties (for example, absorption capacity and/or biodegradability, etc.) of the resulting polymer material can be stably secured.

In another example, the polymer material may comprise a polysaccharide component including a first polysaccharide containing a modified monosaccharide unitary body (e.g., a modified monosaccharide unitary body of Formula 2 above) containing the functional group of Formula 1 above and a second polysaccharide containing a modified monosaccharide unitary body (e.g., a modified monosaccharide unitary body of Formula 4 above) containing the functional group of Formula 3 above.

In this case, the lower limit of the substitutional rate of the functional group of Formula 1 above in the first polysaccharide may be 10%, 15%, 20%, 25%, 30%, 35%, 40%, 45%, 50%, 55%, 60%, 65%, 70%, 75%, 80%, 85%, 90%, or 95% or more or so, and the upper limit thereof may be 300%, 295%, 290%, 285%, 280%, 275%, 270%, 265%, 260%, 255%, 250%, 245%, 240%, 235%, 230%, 225%, 220%, 215%, 210%, 205%, 200%, 195%, 190%, 185 %, 180%, 175%, 170%, 165%, 160%, 155%, 150%, 145%, 140%, 135%, 130%, 125%, 120%, 115%, 110%, 105% or 100% or so. The substitutional rate may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. Within the range of the substitutional rate, cross-linking of the polysaccharide can proceed effectively, and the physical properties (for example, absorption capacity and/or biodegradability, etc.) of the resulting polymer material can be stably secured.

In the above case, the lower limit of the substitutional rate of the functional group of Formula 3 above in the second polysaccharide may be 10%, 15%, 20%, 25%, 30%, 35%, 40%, 45%, 50%, 55%, 60%, 65%, 70%, 75%, 80%, 85%, 90%, 95%, 100%, 105%, 110%, 115%, or 120% or so, and the upper limit thereof may be 300%, 295%, 290%, 285%, 280%, 275%, 270%, 265%, 260%, 255%, 250%, 245%, 240%, 235%, 230%, 225%, 220%, 215%, 210%, 205%, 200%, 195%, 190%, 185%, 180%, 175%, 170%, 165%, 160%, 155%, 150%, 145%, 140%, 135%, 130%, 125%, 120%, 115%, 110%, 105%, or 100% or so. The substitutional rate may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. Within the range of the substitutional rate, cross-linking of the polysaccharide can proceed effectively, and the physical properties (for example, absorption capacity and/or biodegradability, etc.) of the resulting polymer material can be stably secured.

In the above case, the upper limit of the weight ratio of the second polysaccharide relative to 100 parts by weight of the first polysaccharide in the polysaccharide component may be 4000 parts by weight, 3800 parts by weight, 3600 parts by weight, 3400 parts by weight, 3200 parts by weight, 3000 parts by weight, 2800 parts by weight, 2600 parts by weight, 2400 parts by weight, 2200 parts by weight, 2000 parts by weight, 1800 parts by weight, 1600 parts by weight, 1400 parts by weight, 1200 parts by weight, 1000 parts by weight, 950 parts by weight, 900 parts by weight, 850 parts by weight, 800 parts by weight, 750 parts by weight, 700 parts by weight, 650 parts by weight, 600 parts by weight, 550 parts by weight, 500 parts by weight, 450 parts by weight, 400 parts by weight, 350 parts by weight, 300 parts by weight, 250 parts by weight, 200 parts by weight, 150 parts by weight, 100 parts by weight, 90 parts by weight, 80 parts by weight, 70 parts by weight, 60 parts by weight, 50 parts by weight, 40 parts by weight, 30 parts by weight, or 20 parts by weight or so, and the lower limit thereof may be 2800 parts by weight, 2600 parts by weight, 2400 parts by weight, 2200 parts by weight, 2000 parts by weight, 1800 parts by weight, 1600 parts by weight, 1400 parts by weight, 1200 parts by weight, 1000 parts by weight, 950 parts by weight, 900 parts by weight, 850 parts by weight, 800 parts by weight, 750 parts by weight, 700 parts by weight, 650 parts by weight, 600 parts by weight, 550 parts by weight, 500 parts by weight, 450 parts by weight, 400 parts by weight, 350 parts by weight, 300 parts by weight, 250 parts by weight parts, 200 parts by weight, 150 parts by weight, 100 parts by weight, 90 parts by weight, 80 parts by weight, 70 parts by weight, 60 parts by weight, 50 parts by weight, 40 parts by weight, 30 parts by weight, 20 parts by weight, 15 parts by weight, or 10 parts by weight or so. The weight ratio may be in a range of more than or equal to, or more than any one of the above-described lower limits; a range of less than or equal to, or less than any one of the above-described upper limits; or a range of less than or equal to, or less than any one of the above-described upper limits while being more than or equal to, or more than any one of the above-described lower limits. Within the range of

the ratio, cross-linking of the polysaccharide can proceed effectively, and the physical properties (for example, absorption capacity and/or biodegradability, etc.) of the resulting polymer material can be stably secured.

The polymer material, or the polymer such as the polysaccharide component may have an appropriate level of molecular weight. For example, in one example, the polymer material or polymer may have a weight average molecular weight (Mw) in a range of about 500 g/mol to 1,000,000 g/mol. Within such a range, the desired sufficient degree of cross-linking can be imparted to the polymer material when necessary, and the obtained polymer material can stably exhibit the desired properties (biodegradability and absorption capacity, etc.).

In one example, the polysaccharide may be included in a cross-linked state within the polymer material. The cross-linking of such a polysaccharide can be achieved through double bonds present in the functional group (for example, the functional group of Formula 1 above). The cross-linking of such a polysaccharide may be achieved by itself through the double bonds of the polysaccharide, or may be achieved through the application of a so-called cross-linking agent.

In this instance, the type of the applicable cross-linking agent is not particularly limited. For example, in the production of cross-linked polyacrylic acid, which is usually applied as the SAP, a cross-linking agent applied as a so-called internal cross-linking agent, or a cross-linking agent applied as an external cross-linking agent may be applied, or both may also be applied.

As such a cross-linking agent, polyethylene glycol diacrylate, N,N'-methylenebisacrylamide, trimethylolpropane tri(meth)acrylate, ethylene glycol di(meth)acrylate, polyethylene glycol (meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexane diol (meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin and/or ethylene carbonate, and the like are known, and in the present application, if necessary, an appropriate cross-linking agent may be selected from among the known cross-linking agents, and used.

When such a cross-linking agent is applied, the ratio is not particularly limited, and an appropriate ratio may be selected in consideration of the desired degree of cross-linking. For example, such a cross-linking agent may not be used, or may be used in the ratio to be described below.

In one example, the polysaccharide may be included in the polymer material in a self-cross-linked state. In this specification, the term self-cross-linking means a case where the polysaccharide component is cross-linked without using a separate cross-linking agent, or a case where the cross-linking is performed using a minimal cross-linking agent, which relates to the above-described primary cross-linking. That is, the polysaccharide component after the self-cross-linking is performed may be further cross-linked. This further cross-linking may be, for example, a surface treatment of the so-called self-cross-linked polysaccharide component.

Through cross-linking in this way, it is possible to provide a polymer material having excellent absorption capacity and biodegradability simultaneously. In the case of general polysaccharide components, they have low cross-linking efficiency, so that efficient cross-linking is not achieved even when cross-linkable functional groups are introduced, but in the case of the present application, the self-cross-linking can be effectively performed through application of the specific polysaccharide component as described above.

The amount of cross-linking agent in the use of the minimum cross-linking agent is not particularly limited if it is adjusted to a level that does not adversely affect biodegradability, and for example, the upper limit of the ratio of the cross-linking agent relative to 100 parts by weight of the polysaccharide component may be 10 parts by weight, 9 parts by weight, 8 parts by weight, 7 parts by weight, 6 parts by weight, 5 parts by weight, 4 parts by weight, 3 parts by weight, 2 parts by weight, 1 part by weight, 0.5 parts by weight, 0.1 parts by weight, 0.05 parts by weight parts, 0.01 parts by weight, 0.005 parts by weight, or 0.001 parts by weight or so, and the lower limit thereof may be 0 parts by weight or so. The ratio of the cross-linking agent may be in a range of less than or equal to, or less than any one of the above-described upper limits; or a range of less than or equal to, or less than any one of the above-described upper limits while being more than or equal to, or more than any one of the above-described lower limits.

The method of performing the self-cross-linking is not particularly limited. The self-cross-linking may be performed, for example, in the presence of an oxidizing agent such as ammonium persulfate, or other initiators.

In the cross-linking environment, cross-linking, or polymerization by the functional group of Formula 1 present in the polysaccharide component, and cross-linking or polymerization, or grafting by the radicals generated by oxidation by the oxidizing agent and the functional group of Formula 1 may proceed to perform cross-linking.

The polymer material can be used for various applications by exhibiting excellent absorptivity and biodegradability simultaneously.

For example, the polymer material may be used as sanitary products such as diapers or sanitary napkins, or absorbent materials applied to other applications requiring absorption. If necessary, further cross-linking, surface treatment, or physical grinding processes may also be performed on the polymer material to increase the efficiency for use as the sanitary products or absorbent materials.

Therefore, the present application relates to an absorbent material or sanitary product (e.g. diapers, sanitary

napkins, etc.) comprising the polymer material.

The specific method of forming the absorbent material or sanitary product by applying the polymer material is not particularly limited, and for example, the method of forming the absorbent material or sanitary product by applying the existing SAP may be used in the same manner.

## Advantageous Effects

The present application can provide a polymer material having excellent biodegradability and absorption capacity, and a use thereof.

## Description of Drawings

Figures 1 to 3 are views showing $^1$H NMR analysis results of the polysaccharides prepared in Preparation Examples.

## Mode for Invention

Hereinafter, the present application will be described in detail through examples and comparative examples, but the scope of the present application is not limited by the following examples.

### 1. Evaluation of centrifuge retention capacity (CRC)

Centrifuge retention capacity (CRC) was measured according to EDANA (European Disposables and Nonwovens Association) WSP 241.3. About 0.2 g ($W_0$) of a sample (polymer material) was placed in a non-woven bag, sealed, and then submerged in a physiological saline solution. As the physiological saline solution, an aqueous NaCl solution with a concentration of 0.9 wt% was used. The submerged state was maintained for 30 minutes or so, water was removed from the bag for 3 minutes under a condition of 250 G using a centrifuge, and then the mass (g, $W_2$) of the bag was measured. The same operation was performed on the same non-woven bag without the sample, and the mass (g, $W_1$) was measured.

The CRC (g/g) was calculated by substituting the measurement results into Equation A below.

The evaluation was conducted under constant temperature and humidity conditions (23 ± 1°C, relative humidity: 50 ± 10%).

[Equation A]

$$CRC\ (g/g) = \{[W_2(g) - W_1(g)]/W_0(g)\} - 1$$

### 2. Measurement of biodegradability

Biodegradability was measured in the manner specified in KS M ISO 14851 standard. The biodegradability appears differently depending on the measurement method. For example, the KS M ISO 14855-1 is a method of measuring biodegradability through carbon dioxide under a composting condition, where a higher level of biodegradability is measured for the same material compared to other measurement methods, but in the actual environment, it does not represent the biodegradability of the material well. The KS M ISO 14851 standard is a method of measuring aerobic biodegradability of a polymer material in an aqueous solution medium (measurement of oxygen consumption by a closed respiratory system), where the biodegradability of this method well reflects the biodegradation characteristics of the material in the actual environment. The method of measuring biodegradability according to the KS M ISO 14851 standard is known.

### Preparation Example 1.

A polysaccharide (modified starch) (Compound A) containing a modified monosaccharide unitary body of Formula A below was prepared in the following manner. The modified monosaccharide unitary body of Formula A below is a monosaccharide unitary body into which a maleic acid group (a substituent where $M_1$ in Formula 1 is a hydrogen atom) is introduced.

[Formula A]

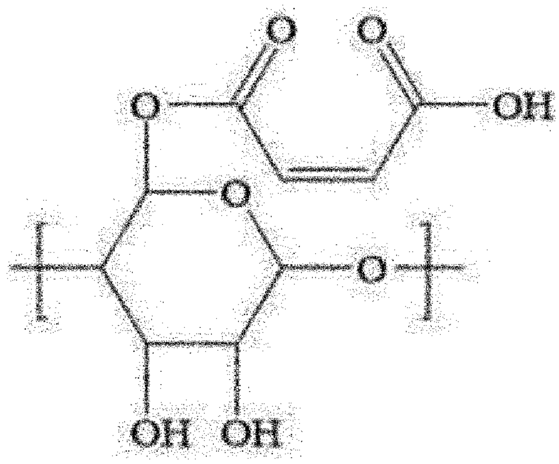

20 g of starch and 50 mL of water were added to a 500 mL RBF (Round Bottom Flask), stirred at room temperature (about 25°C), and then 50 mL of a 2.0 M NaOH solution was added thereto. As the starch, potato starch was used. The mixture was further stirred for 2 hours to be gelatinized, about 100 g of maleic anhydride was added thereto, and reacted at 65°C for 5 hours or so. After completion of the reaction, the temperature was lowered to room temperature (about 25°C), and acetone was added to generate a precipitate. The precipitate was recovered and dried in a vacuum drying oven at 40°C for one day to obtain a solid target product (Compound A). The substitutional rate of the obtained target product (Compound A) can be obtained through a $^1$H NMR analysis. The $^1$H NMR analysis is performed at room temperature (about 25°C) using a $^1$H NMR spectrometer including a Varian Unity Inova (500 MHz) spectrometer with a triple resonance 5 mm probe. In the $^1$H NMR analysis, Bruker's Avance Neo instrument was used. 50 mg of the obtained target product (Compound A) and 200 mg of 30% DCl in $D_2O$ solution are mixed, stirred at 50°C for 1 hour or so to induce a hydrolysis reaction, and then the $^1$H NMR analysis can be performed. The substitutional rate of the maleic acid group to the starch was confirmed by the $^1$H NMR analysis. Figure 1 shows the results of $^1$H NMR analysis performed on Compound A above, and the substitutional rate calculated based on this was about 98% or so.

**Preparation Example 2.**

Starch (Compound B) containing a modified monosaccharide unitary body of Formula B below was prepared in the following manner. The modified monosaccharide unit of Formula B below is a monosaccharide unitary body into which a functional group (substituent, wherein $M_2$ in Formula 3 is sodium and $L_2$ is a methylidene group) derived from sodium acetate is introduced.

[Formula B]

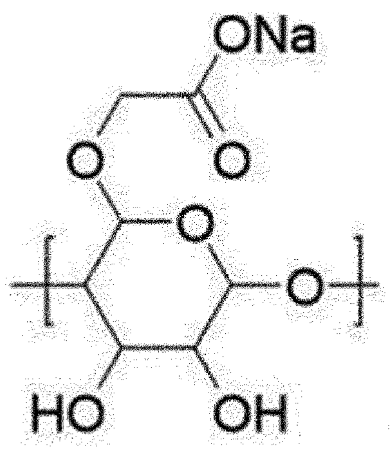

20 g of starch and 50 mL of water were added to a 500 mL RBF (Round Bottom Flask), stirred at room temperature (about 25°C), and then 50 mL of a 2.0 M NaOH solution was added thereto. As the starch, the same starch as in Preparation Example 1 was used. The mixture was further stirred for 2 hours to be gelatinized, about 100 g of sodium monochloroacetate was added thereto, and reacted at 65°C for 5 hours or so. After completion of the reaction, the temperature was

lowered to room temperature (about 25°C), and acetone was added to generate a precipitate. The precipitate was recovered and dried in a vacuum drying oven at 40°C for one day to obtain a solid target product (Compound B).

The results of the $^1$H NMR analysis performed on the target product (Compound B) in the same manner as Preparation Example 1 are shown in Figure 2. The substitutional rate confirmed from the results in Figure 2 was about 120% or so.

**Preparation Example 3.**

Starch (compound C) simultaneously containing the modified monosaccharide unitary bodies represented by Formulas A and B of Preparation Examples 1 and 2 was prepared in the following manner. 20 g of starch and 50 mL of water were added to a 500 mL RBF (Round Bottom Flask), stirred at room temperature (about 25°C), and then 50 mL of a 2.0 M NaOH solution was added thereto. As the starch, the same type as in Preparation Example 1 was used. The mixture was further stirred for 2 hours to be gelatinized, and about 14 g of sodium monochloroacetate and about 10 g of maleic anhydride were added thereto, and reacted at 65°C for 5 hours or so. After completion of the reaction, the temperature was lowered to room temperature (about 25°C), and acetone was added to generate a precipitate. The precipitate was recovered and dried in a vacuum drying oven at 40°C for one day to obtain a solid target product (Compound C). The results of the $^1$H NMR analysis performed on the target product (Compound C) in the same manner as Preparation Example 1 are shown in Figure 3. The substitutional rate of the maleic acid group of the target product (Compound C) confirmed from the analysis results in Figure 3 was about 37% or so, and the substitutional rate of the sodium acetate-derived functional group was about 68% or so.

**Example 1.**

50 mL of distilled water, 2.5 g of the compound of Preparation Example 1 (Compound A), and 0.5 g of the compound of Preparation Example 2 (Compound B) were introduced into a 250 mL RBF (Round Bottom Flask), and the mixture was stirred in an oil bath at 35°C for 30 minutes or more, whereby Compounds A and B were sufficiently dissolved in the distilled water. Then, an initiator was introduced thereto. As the initiator, about 0.03 g of ammonium persulfate was introduced. After introducing the initiator, the mixture was further stirred at a temperature of 70°C or so for 4 hours or so to proceed cross-linking between Compounds A and B. After cross-linking, ethanol was introduced to precipitate a polymer material containing the cross-linked polymer, and then dried overnight in a vacuum drying oven at 40°C after filtering to obtain a target polymer material.

**Examples 2 to 4 and Comparative Examples 1 and 2**

Polymers were prepared in the same manner as in Example 1, except that the amounts of the compounds introduced for polymerization were changed as in Table 1 below. In the table below, the unit of content is g.

[Table 1]

| | Example | | | | | Comparative Example | |
|---|---|---|---|---|---|---|---|
| | 1 | 2 | 3 | 4 | 5 | 1 | 2 |
| Compound A | 2.5 | 2 | 0.5 | 0.1 | - | 3 | - |
| Compound B | 0.5 | 1 | 1.5 | 2.9 | - | - | 3 |
| Compound C | - | - | - | - | 3 | - | - |

**Comparative Example 3**

50 mL of distilled water, 2.5 g of the compound of Preparation Example 1 (Compound A), 0.5 g of the compound of Preparation Example 2 (Compound B), 6.8 mg of polyethylene glycol diacrylate (number average molecular weight: 575 g/mol), 0.24 mg of bis(2,4,6-trimethylbenzoyl)-phenylphosphine oxide, and 3.6 mg of sodium persulfate were introduced into a 250 mL RBF (Round Bottom Flask), and the mixture was stirred in an oil bath at 35°C for 30 minutes or more, whereby Compounds A and B were sufficiently dissolved in the distilled water. In a UV chamber at 80°C, the mixture was irradiated with ultraviolet rays for 60 seconds (irradiation dose: 10 mV/cm$^2$), and then maintained for about 2 minutes to proceed cross-linking. After cross-linking, ethanol was introduced to precipitate a polymer material containing the cross-linked polymer, and then dried overnight in a vacuum drying oven at 40°C after filtering to obtain a target polymer material.

The CRC and biodegradability measured for the polymers of the examples and comparative examples were summarized in Table 2 below and described.

[Table 2]

| | Example | | | | | Comparative Example | | |
|---|---|---|---|---|---|---|---|---|
| | 1 | 2 | 3 | 4 | 5 | 1 | 2 | 3 |
| CRC(g/g) | 16.1 | 16.8 | 17.5 | 16.0 | 17.1 | 11.3 | 2.3 | 15.1 |
| Biodegradability (%) | 86 | 88 | 74 | 70 | 80 | 85 | 52 | 59 |

As in the results of Table 2, the polymer materials according to Examples of the present application simultaneously exhibited excellent absorption properties and biodegradability. Comparative Example 1 was a case where only Compound A was applied, and in this case, the biodegradability was secured to a certain extent, but the absorption characteristic was greatly reduced. Comparative Example 2 was a case where only Compound B was applied, and both absorption capacity and biodegradability were reduced. Comparative Example 3 was a case where the same materials as those of Example 1 were used, but a method using an acrylate-based cross-linking agent was applied instead of a self-cross-linking method, and in this case, absorption capacity was secured, but biodegradability was greatly reduced.

## Claims

1. A polymer material comprising a polysaccharide component, wherein

   a centrifuge retention capacity according to EDANA method WSP 241.3 is 13 g/g or more, and
   a biodegradability according to KS M ISO 14851 standard is 70% or more.

2. The polymer material according to claim 1, wherein the polysaccharide component comprises a polysaccharide having a modified monosaccharide unitary body.

3. The polymer material according to claim 2, wherein the modified monosaccharide unitary body comprises a functional group of Formula 1 below:

[Formula 1]

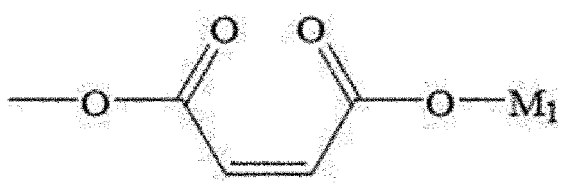

wherein, $M_1$ is hydrogen or a metal, and when $M_1$ is the metal, the O-$M_1$ bond is an ionic bond, and the carbon-carbon double bond in Formula above may form a cross-linked structure.

4. The polymer material according to claim 3, wherein the substitutional rate of the functional group of Formula 1 in the polysaccharide is in a range of 10% to 300%.

5. The polymer material according to claim 2, wherein the modified monosaccharide unitary body is represented by Formula 2 below:

[Formula 2]

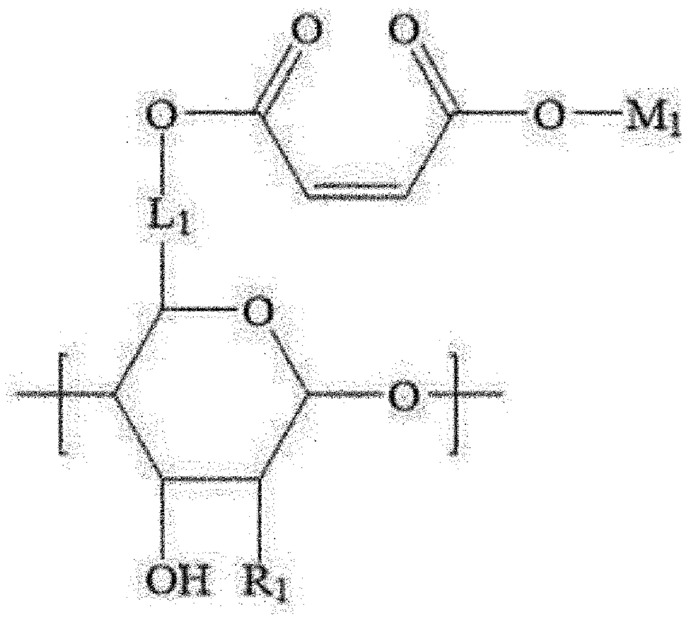

wherein, $R_1$ is a hydroxy group, an amino group, or an alkylcarbonylamino group, $L_1$ is an alkylene group or an alkylidene group, $M_1$ is hydrogen or a metal, and when $M_1$ is the metal, the O-$M_1$ bond is an ionic bond, and the carbon-carbon double bond in Formula above may react to form a cross-linked structure.

6. The polymer material according to claim 2, wherein the modified monosaccharide unitary body comprises a functional group of Formula 3 below:

[Formula 3]

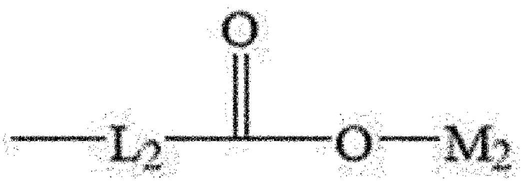

wherein, $L_2$ is an alkylene group or an alkylidene group, and $M_2$ is hydrogen or a metal, and when $M_2$ is the metal, the O-$M_2$ bond is an ionic bond.

7. The polymer material according to claim 6, wherein the substitutional rate of the functional group of Formula 3 in the polysaccharide is in a range of 10% to 300%.

8. The polymer material according to claim 2, wherein the modified monosaccharide unitary body is represented by Formula 4 below:

[Formula 4]

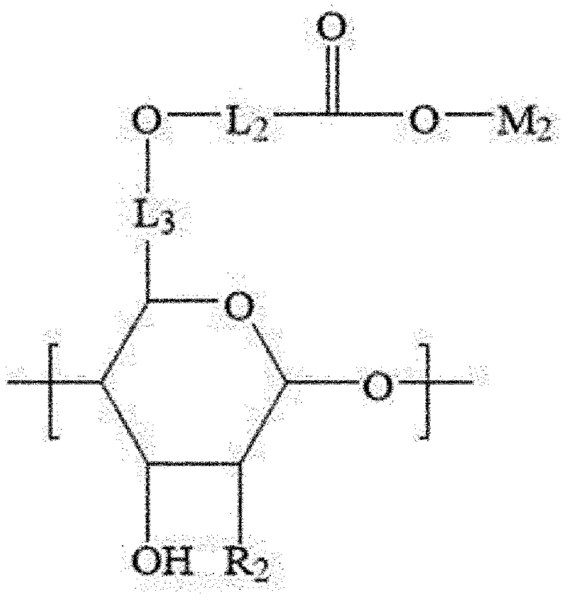

wherein, $R_2$ is a hydroxy group, an amino group, or an alkylcarbonylamino group, $L_2$ and $L^3$ are each independently an alkylene group or an alkylidene group, $M_2$ is hydrogen or a metal, and when $M_2$ is the metal, the O-$M_2$ bond is an ionic bond.

**9.** The polymer material according to claim 1, wherein the polysaccharide component comprises a polysaccharide including a modified monosaccharide unitary body having a functional group of Formula 1 below and a modified monosaccharide unitary body having a functional group of Formula 3 below:

[Formula 1]

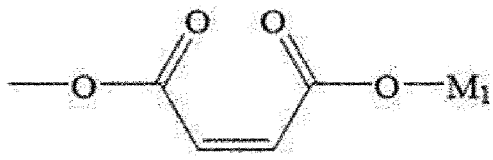

[Formula 3]

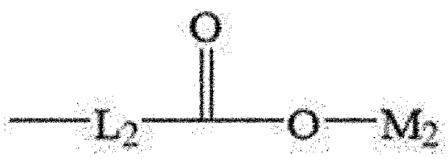

wherein, $M_1$ in Formula 1 is hydrogen or a metal, and when $M_1$ is the metal, the O-$M_1$ bond is an ionic bond, and the carbon-carbon double bond in Formula above may react to form a cross-linked structure, and $L_2$ in Formula 3 is an alkylene group or an alkylidene group, and $M_2$ is hydrogen or a metal, and when $M_2$ is the metal, the O-$M_2$ bond is an ionic bond.

**10.** The polymer material according to claim 9, wherein the substitutional rate of the functional group of Formula 1 in the polysaccharide is in a range of 10% to 300%, and the substitutional rate of the functional group of Formula 3 is in a range of 10% to 300%.

**11.** The polymer material according to claim 1, wherein the polysaccharide component comprises a first polysaccharide containing a modified monosaccharide unitary body having a functional group of Formula 1 below and a second polysaccharide containing a modified monosaccharide unitary body having a functional group of Formula 3 below:

[Formula 1]

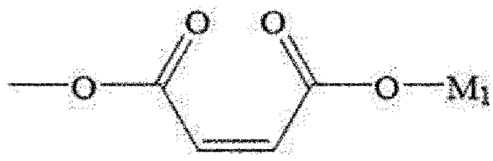

[Formula 3]

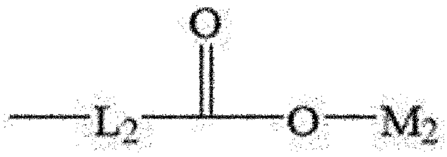

wherein, $M_1$ in Formula 1 is hydrogen or a metal, and when $M_1$ is the metal, the O-$M_1$ bond is an ionic bond, and the carbon-carbon double bond in Formula above may react to form a cross-linked structure, and $L_2$ in Formula 3 is an alkylene group or an alkylidene group, and $M_2$ is hydrogen or a metal, and when $M_2$ is the metal, the O-$M_2$ bond is an ionic bond.

**12.** The polymer material according to claim 11, wherein the substitutional rate of the functional group of Formula 1 in the first polysaccharide is in a range of 10% to 300%, and the substitutional rate of the functional group of Formula 3 in the second polysaccharide is in a range of 10% to 300%.

**13.** The polymer material according to claim 11, comprising 10 to 4000 parts by weight of the second polysaccharide relative to 100 parts by weight of the first polysaccharide.

**14.** The polymer material according to claim 1, wherein the polysaccharide is included in a cross-linked state.

**15.** The polymer material according to claim 1, wherein the polysaccharide is included in a self-cross-linked state.

**16.** The polymer material according to claim 1, comprising no cross-linking agent, or comprising 10 parts by weight or less of a cross-linking agent relative to 100 parts by weight of the polysaccharide component.

**17.** An absorbent material comprising the polymer material of any one of claims 1 to 16.

**18.** A sanitary article comprising the polymer material of any one of claims 1 to 16.

[Figure 1]

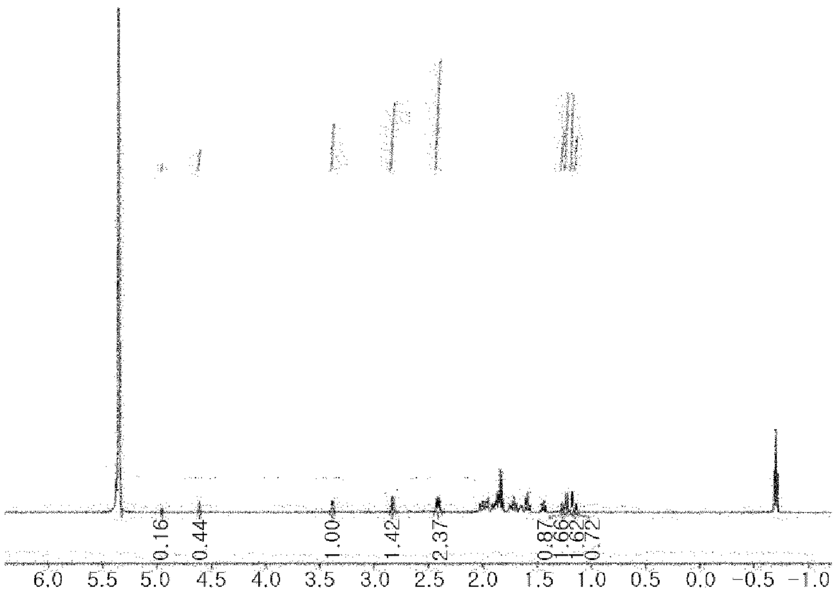
0.16
0.44
1.00
1.42
2.37
0.87
1.66
1.62
0.72
6.0
5.5
5.0
4.5
4.0
3.5
3.0
2.5
2.0
1.5
1.0
0.5
0.0
−0.5
−1.0

[Figure 2]

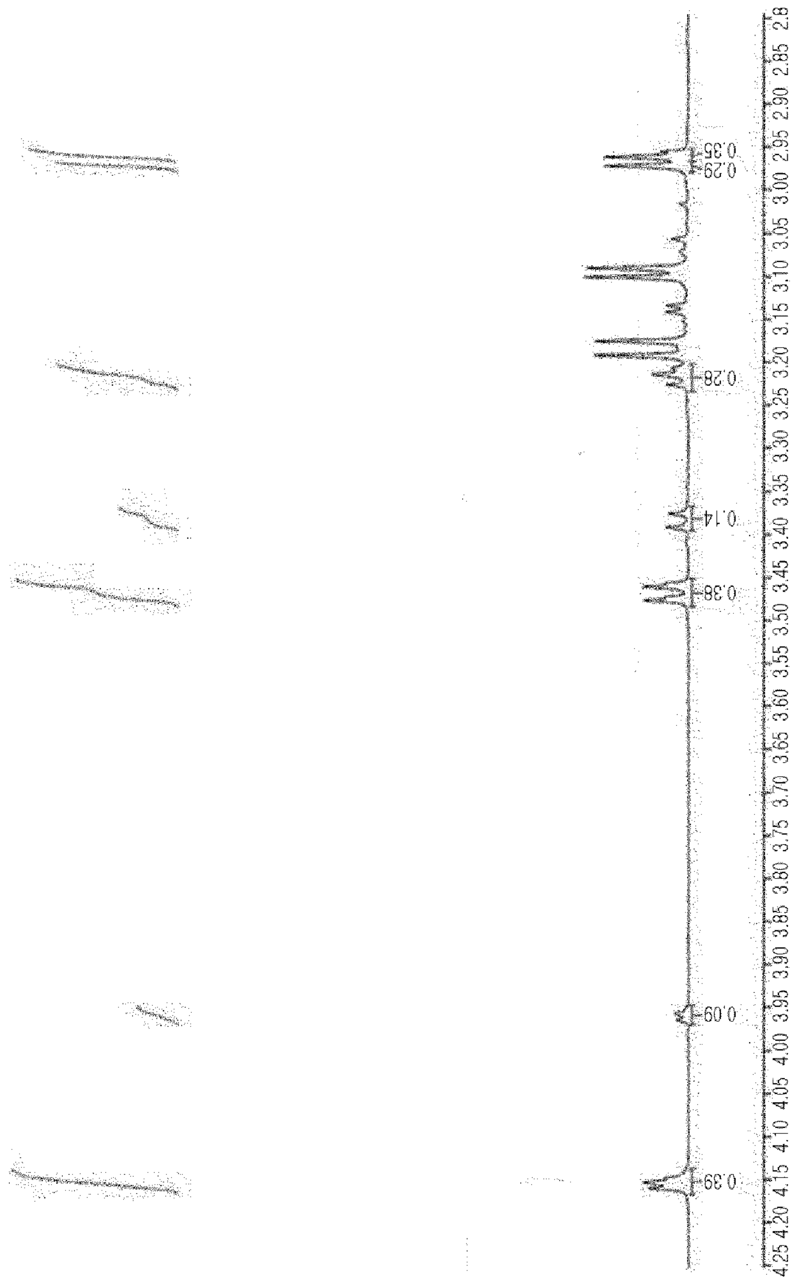

[Figure 3]

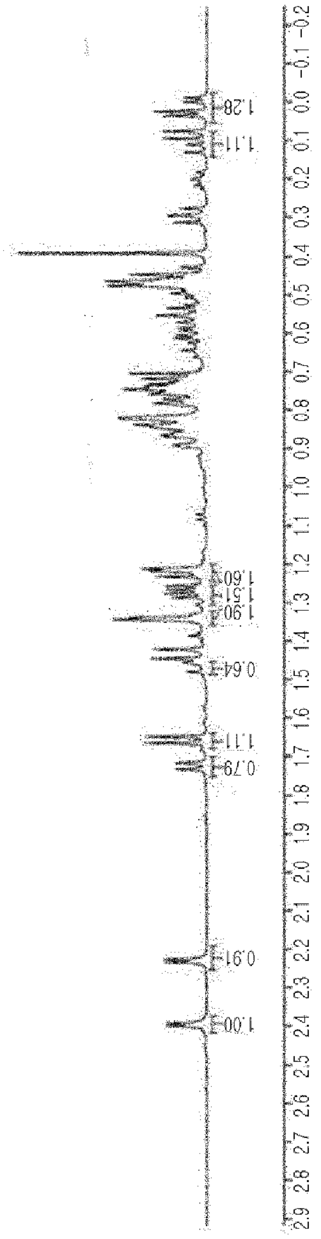

**INTERNATIONAL SEARCH REPORT**

International application No. **PCT/KR2023/006832**

**A. CLASSIFICATION OF SUBJECT MATTER**

**C08L 5/00**(2006.01)i; **C08B 37/00**(2006.01)i; **C08J 3/075**(2006.01)i; **A61L 15/28**(2006.01)i; **A61L 15/60**(2006.01)i

According to International Patent Classification (IPC) or to both national classification and IPC

**B. FIELDS SEARCHED**

Minimum documentation searched (classification system followed by classification symbols)

C08L 5/00(2006.01); A61L 15/60(2006.01); B01D 69/12(2006.01); C08B 11/12(2006.01); C08B 37/00(2006.01); C08B 37/02(2006.01); C08F 251/00(2006.01); C08L 1/26(2006.01); C08L 3/08(2006.01); D21H 11/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), PubChem, Google & keywords: 다당류(polysaccharide), 관능기(functional group), 흡수 재료(absorbent material)

**C. DOCUMENTS CONSIDERED TO BE RELEVANT**

| Category* | Citation of document, with indication, where appropriate, of the relevant passages | Relevant to claim No. |
|---|---|---|
| X | KR 10-2022-0041752 A (LG CHEM, LTD.) 01 April 2022 (2022-04-01) See claims 1, 8 and 12; paragraphs [0072], [0169], [0088]-[0093], [0304] and [0318]; table 1; and example 3. | 1-5,14,16-18 |
| Y | | 9-13,15 |
| A | | 6-8 |
| X | KR 10-2001-0105311 A (SCA HYGIENE PRODUCTS ZEIST B.V.) 28 November 2001 (2001-11-28) See claims 1 and 2; paragraphs [0014], [0029] and [0030]; and example 3. | 1,2,6-8,14,16-18 |
| Y | | 9-13 |
| Y | KR 10-2016-0020524 A (ASAHI KASEI FIBERS CORPORATION) 23 February 2016 (2016-02-23) See paragraph [0215]. | 15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 August 2023** | **24 August 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2023/006832

C. DOCUMENTS CONSIDERED TO BE RELEVANT

| Category* | Citation of document, with indication, where appropriate, of the relevant passages | Relevant to claim No. |
|---|---|---|
| A | US 5247072 A (NING, X. et al.) 21 September 1993 (1993-09-21)<br>See entire document. | 1-18 |
| A | US 5736595 A (GÜNTHER, U. et al.) 07 April 1998 (1998-04-07)<br>See entire document. | 1-18 |

Form PCT/ISA/210 (second sheet) (July 2022)

**INTERNATIONAL SEARCH REPORT**
**Information on patent family members**

International application No.
**PCT/KR2023/006832**

| Patent document cited in search report | Publication date (day/month/year) | Patent family member(s) | Publication date (day/month/year) |
|---|---|---|---|
| KR 10-2022-0041752 A | 01 April 2022 | CN 114901714 A | 12 August 2022 |
| | | EP 4063413 A1 | 28 September 2022 |
| | | JP 2023-509980 A | 10 March 2023 |
| | | US 2023-0087087 A1 | 23 March 2023 |
| | | WO 2022-065843 A1 | 31 March 2022 |
| KR 10-2001-0105311 A | 28 November 2001 | AT 483480 T | 15 October 2010 |
| | | AU 1897500 A | 03 July 2000 |
| | | AU 767859 B2 | 27 November 2003 |
| | | BR 9916235 A | 04 September 2001 |
| | | CA 2356849 A1 | 22 June 2000 |
| | | CA 2356849 C | 03 June 2008 |
| | | EP 1140229 A1 | 10 October 2001 |
| | | EP 1140229 B1 | 06 October 2010 |
| | | ES 2353083 T3 | 25 February 2011 |
| | | JP 2002-532573 A | 02 October 2002 |
| | | MX PA01006098 A | 27 March 2002 |
| | | NZ 512254 A | 28 November 2003 |
| | | PL 348821 A1 | 17 June 2002 |
| | | RU 2227753 C2 | 27 April 2004 |
| | | SK 7982001 A3 | 05 February 2002 |
| | | TN SN99243 A1 | 31 December 2001 |
| | | US 2004-0236016 A1 | 25 November 2004 |
| | | US 6765042 B1 | 20 July 2004 |
| | | WO 00-35504 A1 | 22 June 2000 |
| KR 10-2016-0020524 A | 23 February 2016 | CA 2917792 A1 | 22 January 2015 |
| | | CA 2917792 C | 20 March 2018 |
| | | CN 105431587 A | 23 March 2016 |
| | | CN 105431587 B | 30 July 2019 |
| | | CN 110016833 A | 16 July 2019 |
| | | EP 3023542 A1 | 25 May 2016 |
| | | EP 3023542 A4 | 22 June 2016 |
| | | EP 3023542 B1 | 30 January 2019 |
| | | JP 6097394 B2 | 15 March 2017 |
| | | KR 10-2018-0113639 A | 16 October 2018 |
| | | US 10323354 B2 | 18 June 2019 |
| | | US 2016-0177512 A1 | 23 June 2016 |
| | | US 2019-0257032 A1 | 22 August 2019 |
| | | WO 2015-008868 A1 | 22 January 2015 |
| US 5247072 A | 21 September 1993 | AU 2729192 A | 29 April 1993 |
| | | AU 658455 B2 | 13 April 1995 |
| | | BR 9204036 A | 04 May 1993 |
| | | CA 2073292 A1 | 26 April 1993 |
| | | CA 2073292 C | 29 June 2004 |
| | | DE 69223587 T2 | 23 July 1998 |
| | | DE 69223587 T3 | 14 August 2002 |
| | | EP 0538904 A2 | 28 April 1993 |
| | | EP 0538904 A3 | 13 October 1993 |
| | | EP 0538904 B1 | 17 December 1997 |
| | | EP 0538904 B2 | 10 October 2001 |
| | | ES 2111032 T3 | 01 March 1998 |

Form PCT/ISA/210 (patent family annex) (July 2022)

**INTERNATIONAL SEARCH REPORT**
**Information on patent family members**

International application No.
**PCT/KR2023/006832**

| Patent document cited in search report | Publication date (day/month/year) | Patent family member(s) | Publication date (day/month/year) |
|---|---|---|---|
| | | ES 2111032 T5 | 16 December 2001 |
| | | JP 05-214001 A | 24 August 1993 |
| | | JP 3249201 B2 | 21 January 2002 |
| | | KR 10-0238386 B1 | 15 January 2000 |
| | | KR 10-1993-0012829 A | 21 July 1993 |
| | | MX 9205548 A | 01 April 1993 |
| | | TW 268899 B | 21 January 1996 |
| | | US 5247072 A | 21 September 1993 |
| | | ZA 927461 B | 13 April 1993 |
| US 5736595 A | 07 April 1998 | AU 4029693 A | 21 November 1994 |
| | | AU 677581 B2 | 01 May 1997 |
| | | BG 100175 A | 31 January 1997 |
| | | BG 62857 B1 | 29 September 2000 |
| | | CA 2161904 A1 | 10 November 1994 |
| | | CA 2161904 C | 30 January 2001 |
| | | DE 4206850 A1 | 09 September 1993 |
| | | DE 4206850 C2 | 29 August 1996 |
| | | EP 0701587 A1 | 20 March 1996 |
| | | EP 0701587 B1 | 06 August 1997 |
| | | ES 2107021 T3 | 16 November 1997 |
| | | FI 955226 A | 01 November 1995 |
| | | JP 08-509511 A | 08 October 1996 |
| | | KR 10-0292923 B1 | 15 June 2001 |
| | | PL 174749 B1 | 30 September 1998 |
| | | PL 311586 A1 | 19 February 1996 |
| | | RU 2146270 C1 | 10 March 2000 |
| | | SK 131695 A3 | 08 January 1997 |
| | | SK 280641 B6 | 16 May 2000 |
| | | TW 311922 B | 01 August 1997 |
| | | WO 94-25521 A1 | 10 November 1994 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020220062381